# FASCICULE DE BREVET EUROPEEN

(11) **EP 0 896 529 B1**
(45) Date de publication et mention de la délivrance du brevet: **06.03.2002**
(21) Numéro de dépôt: 97919485.9
(22) Date de dépôt: 04.04.1997
(51) Int. Cl.: A61K 9/48, A61K 31/565, A61K 31/57

(54) **MEDICAMENT A BASE DE PROGESTERONE ET D'OESTRADIOL**
ARZNEIMITTEL AUF BASIS VON PROGESTERON UND ÖSTRADIOL
ESTRADIOL AND PROGESTERONE-BASED MEDICAMENT

(30) Priorité: 05.04.1996 FR 9604349
(43) Date de publication de la demande: 17.02.1999
(73) Titulaire: Laboratoires BESINS ISCOVESCO Société anonyme dite :, 75003 Paris (FR)
(72) Inventeur: SALIN-DROUIN, Dominique, F-91370 Verrières-les-Buissons (FR)
(74) Mandataire: Nargolwalla, Cyra
(86) Numéro de dépôt international: FR9700612
(87) Numéro de publication internationale: WO9737642

(56) Documents cités:
- WO-A-95/00125
- US-A- 4 624 665
- US-A- 4 900 734
- CHEMICAL ABSTRACTS, vol. 90, no. 22, 28 Mai 1979 Columbus, Ohio, US; abstract no. 174616d, G.P. D' ONFORIO ET AL.: "ENCAPSULATED MICROCAPSULES" page 390; colonne 1; XP002022220 & INT. J. PHARM., vol. 2, no. 2, 1979, pages 91-99,

## Description

La présente invention concerne un médicament à base d'oestradiol et de progestérone destiné au traitement de la pathologie ménopausique.

On sait en effet que la carence oestrogènique qui se manifeste pendant la période de la ménopause est parfois difficilement supportable par l'organisme féminin en raison notamment du fait qu'elle est ressentie non seulement au niveau du système nerveux central où elle est responsable de manifestations d'ordre neurovégétatif, telles que des bouffées de chaleur, mais également au niveau de la matrice osseuse.

On a proposé par le passé diverses méthodes d'administration de l'oestradiol et notamment une méthode d'administration par voie percutanée. Le médicament objet de la présente invention est administrable quant à lui par voie orale.

On sait également que, dans le cas d'une oestrogénothérapie, il est habituel d'administrer un traitement de progestérone afin d'éviter notamment les risques d'hyperplasie de l'endomètre.

Plutôt que d'imposer au patient une prise séparée de ces deux médicaments on comprendra qu'il est particulièrement intéressant de regrouper ceux-ci dans un seul et même médicament afin d'autoriser leur absorption par une prise unique.

On connaît, notamment par le brevet FR-A-2.408.345 des médicaments à base de progestérone qui sont administrables par voie orale. Dans certains de ces médicaments la progestérone se trouve à l'état micronisé en suspension dans un liquide constitué par de l'huile. Un but de la présente invention est d'inclure à ce médicament une quantité appropriée d'oestradiol.

Une difficulté provient du fait que, pour obtenir une dissolution complète de l'oestradiol dans certaines huiles, et notamment dans l'huile d'arachide, la quantité d'huile nécessaire est telle que la capsule de médicament est alors d'un volume qui rend plus difficile son absorption par l'utilisateur. On peut, bien entendu, diminuer le volume de cette capsule, mais alors la quantité d'huile contenue dans celle-ci est insuffisante pour permettre une dissolution totale de l'oestradiol, si bien que ce dernier risque de recristalliser, ce qui réduit de façon notoire son caractère actif. En effet, il est parfaitement connu que la taille des particules a un effet direct sur l'absorption des molécules lipophiles et donc sur leur activité.

On connaît également par le brevet US-A-4 900 734 des compositions destinées au traitement de la pathologie ménopausique contenant une progestérone à l'état micronisé en suspension dans une huile de type polyinsaturé et de l'oestradiol solubilisé dans cette huile.

La présente invention a pour but de proposer un moyen permettant de délivrer dans une capsule unique de petites dimensions l'oestradiol et la quantité de progestérone qui doit lui être normalement associée.

La présente invention a ainsi pour objet un médicament constitué d'une capsule soluble en milieu biologique contenant une progestérone à l'état micronisé en suspension dans de l'huile, caractérisé en ce que la capsule contient également de l'oestradiol renfermé dans des microsphères également en suspension dans l'huile et qui sont constituées d'au moins un polymère apte à ne pas se dissoudre dans l'huile et à se dissoudre en milieu biologique.

Dans un mode de mise en oeuvre de l'invention ce polymère est un polymère cellulosique, notamment du type commercialisé sous la marque "KLUCEL EFEP". Ce polymère peut également être un polymère acrylique notamment commercialisé sous la marque "EUDRAGIT E100".

De façon particulièrement intéressante la demanderesse a établi, ainsi que montré sur les courbes cinétiques de dissolution représentées ci-après, que la vitesse de dissolution de l'oestradiol contenu dans des microsphères est au moins égale, sinon supérieure, à ce qu'elle est à l'état normal, c'est-à-dire dans un état micronisé (autrement dit un état dans lequel les particules ont des dimensions de l'ordre de 5 µm). Cette propriété est tout particulièrement intéressante dans la mesure où, jusqu'à présent, la mise en microsphères d'une substance active avait pour effet de ralentir la libération de celle-ci.

On décrira ci-après, à titre d'exemples non limitatifs, diverses formes d'exécution de la présente invention, en référence aux dessins annexés sur lesquels :
La figure 1 est un graphique représentant les courbes de la cinétique de dissolution de l'oestradiol respectivement à l'état micronisé, et à l'état de microsphères dans plusieurs polymères.
La figure 2 est un graphique représentant les courbes de dissolution de l'oestradiol inclus dans des microsphères qui respectivement ont été immergées et non immergées dans l'huile.

Suivant l'invention on a réalisé des microsphères d'oestradiol par des moyens, connus de l'état antérieur de la technique sous la dénomination de "nébulisation" (ou également "spray drying"). Suivant cette technique, on a introduit des particules d'oestradiol au sein d'un grand nombre de microsphères réalisées dans trois polymères différents (numérotés II à IV). On a soumis chacune de ces trois séries d'échantillons à une dissolution dans un milieu biologique, ou plus précisément dans un modèle de milieu biologique communément admis par les spécialistes, et qui est constitué d'eau additionnée de 0,3% de laurylsulfate de sodium.

On a prélevé des échantillons au cours de cette cinétique, à des intervalles de temps de 1 minute, 30 minutes et 60 minutes, et l'on a mesuré, par une méthode de chromatographie liquide haute performance, le pourcentage d'oestradiol libéré.

On a représenté sur le tableau ci-après les quantités d'oestradiol libérées au cours de cette cinétique, respectivement sur un échantillon témoin constitué d'oestradiol à l'état micronisé (échantillon I) et des trois échantillons II à IV ci-dessus mentionnés :
- Échantillon II : Mélange à 50% en masse d'un polymère acrylique (Marque déposée EUDRAGIT E100) et d'un polyvinyl pyrrolidone (Marque déposée KOLLIDON 30)
- Échantillon III : Mélange à 50% en masse d'un polymère dérivé cellulosique (Marque déposée KLUCEL EFEP) et d'un polyvinyl pyrrolidone (Marque déposée KOLLIDON 30)
- Échantillon IV : Mélange à 50% en masse d'un polymère acrylique (Marque déposée EUDRAGIT RL 100) et d'un polyvinyl pyrrolidone (Marque déposée KOLLIDON 30)

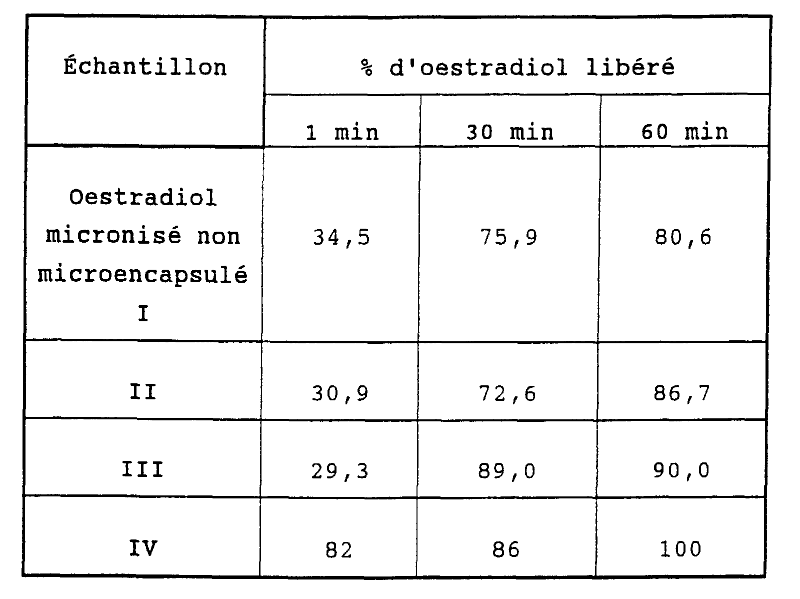

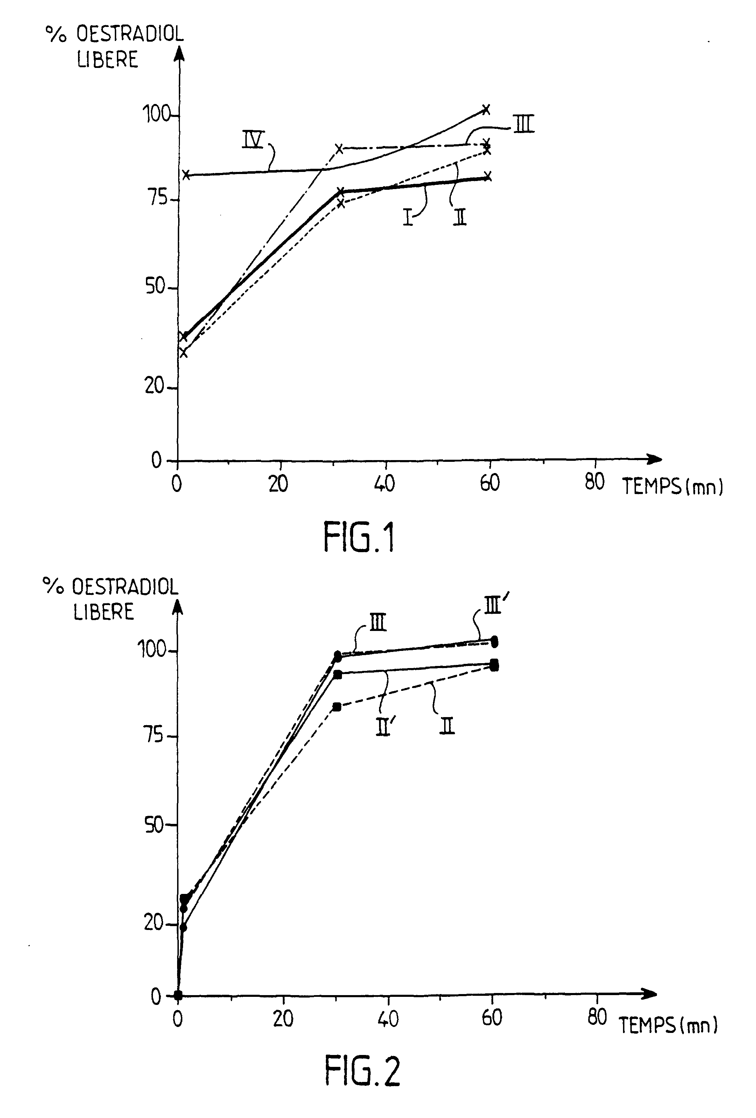

On a représenté sur la figure 1 la variation du pourcentage d'oestradiol libéré en fonction du temps qui est caractéristique des cinétiques de dissolution de ces quatre échantillons.

L'étude de ce tableau, et des courbes correspondantes qui lui sont associées, montre que les vitesses de dissolution de l'oestradiol renfermé dans des microsphères sont au moins égales, et le plus souvent supérieures, à celles de l'oestradiol micronisé seul. Cette particularité est d'une part particulièrement surprenante puisque l'on sait qu'il est admis par les spécialistes en la matière qu'une substance active enrobée dans une microsphère se libère moins rapidement que lorsqu'elle est non enrobée. Elle est de plus intéressante car elle est de nature, dans certains cas, à favoriser l'absorption de la substance active.

On a ensuite effectué des tests afin de vérifier si l'immersion dans l'huile des microsphères, constituées de différents polymères renfermant des particules d'oestradiol avait une influence sur la cinétique de dissolution de ces particules. Pour ce faire on a réalisé des cinétiques à partir d'une part d'échantillons d'oestradiol sous forme de microsphères ayant été immergés dans une quantité donnée d'huile et d'autre part d'échantillons d'oestradiol sous forme de microsphères n'ayant pas subi d'immersion dans l'huile. On a utilisé des échantillons identiques aux échantillons II et III. Sur le graphique de la figure 2 on a affecté de l'indice ' les échantillons qui ont été immergés dans l'huile.

Pour réaliser cette cinétique on a respecté le rapport des quantités d'oestradiol et d'huile en présence. Ainsi dans la formule de médicament 1mg d'oestradiol étant en contact avec 149mg d'huile d'arachide LIPEX 101, afin de conserver ce rapport, on a mis en présence 60mg d'oestradiol, (soit 600mg de microsphères) avec 9.150mg d'huile d'arachide LIPEX 101.

Après un temps de contact de quelques heures, on a éliminé par un lavage à l'hexane, la phase huileuse qui recouvrait les capsules, puis l'on a effectué une filtration. On a choisi l'hexane en tant que solvant de rinçage car il ne dissout pas l'oestradiol ni aucun des constituants qui forment la microsphère.

Une fois sèches, les microsphères ont été récupérées et la cinétique de dissolution a été réalisée. On a comparé ensuite les cinétiques de dissolution effectuées sur les microsphères préalablement immergées dans l'huile aux cinétiques de dissolution réalisées sur des microsphères non traitées. On a représenté sur la figure 2 la variation de pourcentage d'oestradiol libéré en fonction du temps qui est caractéristique des séries d'échantillons. On n'observe pas sur cette figure de différence réellement significative entre des microsphères ayant subi ou non l'immersion dans l'huile.

La présente invention est particulièrement intéressante en ce qu'elle permet, à partir d'un médicament, dont quelques années d'utilisation ont démontré le caractère efficace, (à savoir un médicament formé d'une capsule soluble en milieu biologique contenant de l'huile dans laquelle des particules micronisées de progestérone sont en suspension) d'associer un autre principe actif dont l'efficacité a également été démontrée par l'usage, de façon à constituer un médicament bicomposant présentant à la fois les avantages de l'un et de l'autre sans que l'un des médicaments n'ait une interaction sur l'autre et tout en n'imposant pas au patient utilisateur les contraintes propres à l'administration de deux médicaments séparés.

## Revendications

1. Médicament constitué d'une capsule soluble en milieu biologique contenant une progestérone à l'état micronisé en suspension dans de l'huile, **caractérisé en ce que** la capsule contient également de l'oestradiol renfermé dans des microsphères également en suspension dans l'huile et qui sont constituées d'au moins un polymère apte à ne pas se dissoudre dans l'huile et à se dissoudre en milieu biologique.

2. Médicament suivant la revendication 1, **caractérisé en ce que** ce polymère est un polymère dérivé cellulosique.

3. Médicament suivant la revendication 1, **caractérisé en ce que** ce polymère est un polymère acrylique.

4. Médicament suivant la revendication 1, **caractérisé en ce que** le polymère est un polyvinyl pyrrolidone.

5. Médicament suivant l'une des revendications précédentes, **caractérisé en ce que** l'huile est de l'huile d'arachide.

## Claims

1. Medicament constituted by a capsule soluble in biological milieu containing a progesterone which is micronised in suspension in oil, **characterised in that** the capsule further contains oestradiol enclosed in micro-spheres also in suspension in the oil, and that the capsules are constituted by at least one polymer which does not dissolve in the oil and which dissolves in the biological milieu.

2. Medicament according to claim 1, **characterised in that** the polymer is a polymer of a cellulosic derivative.

3. Medicament according to claim 1, **characterised in that** the polymer is an acrylic polymer.

4. Medicament according to claim 1, **characterised in that** the polymer is a polyvinyl pyrrolidone.

5. Medicament according to any one of the preceding claims, **characterised in that** the oil is peanut oil.

## Patentansprüche

1. Medikament, das aus einer in biologischem Milieu lösbaren Kapsel gebildet ist, enthaltend ein Progesteron im mikronisierten Zustand in Suspension in Öl, **dadurch gekennzeichnet, daß** die Kapsel auch Östradiol enthält, das in Mikrokügelchen eingeschlossen ist, die auch in Suspension im Öl sind und die aus wenigstens einem Polymer gebildet sind, das sich im Öl nicht auflösen und im biologischen Milieu auflösen kann.

2. Medikament nach Anspruch 1, **dadurch gekennzeichnet, daß** dieses Polymer ein aus Zellulose hergeleitetes Polymer ist.

3. Medikament nach Anspruch 1, **dadurch gekennzeichnet, daß** dieses Polymer ein Acrylpolymer ist.

4. Medikament nach Anspruch 1, **dadurch gekennzeichnet, daß** das Polymer ein Polyvinylpyrrolidon ist.

5. Medikament nach einem der vorhergehenden Ansprüche, **dadurch gekennzeichnet, daß** das Öl Erdnußöl ist.
